# EUROPEAN PATENT APPLICATION

(11) **EP 4 331 583 A1**
(43) Date of publication of application: **06.03.2024**
(21) Application number: 22795833.7
(22) Date of filing: 27.04.2022
(51) Int. Cl.: A61K 31/424, A61K 31/205, A61P 3/00

(54) **BLOOD CARNITINE-INCREASING AGENT**

(30) Priority: 28.04.2021 JP 2021076350
(71) Applicant: KOWA COMPANY, LTD., Naka-ku Nagoya-shi Aichi 460-8625 (JP)
(72) Inventor: TANIGAWA, Ryohei, Tokyo 103-8433 (JP); SAITO, Ayumi, Tokyo 103-8433 (JP); IKEGAMI, Kaho, Tokyo 103-8433 (JP)
(74) Representative: Wächtershäuser & Hartz Patentanwaltspartnerschaft mbB
(86) International application number: PCT/JP2022/019018
(87) International publication number: WO 2022/230920

(57) **Abstract**

Provided is a novel preventing and/or treating agent useful for prevention and/or treatment of carnitine deficiency, or a disease for which supplementation of carnitine may have a therapeutic effect. The present invention relates to a medicament for preventing and/or treating carnitine deficiency, or a disease for which supplementation of carnitine may have a therapeutic effect, the medicament comprising a therapeutically effective amount of (R)-2-[3-[[N-(benzoxazol-2-yl)-N-3-(4-methoxyphenoxy)propyl]aminomethyl]phenoxy]butyric acid, a salt thereof, or a solvate thereof.

## Description

### Technical Field

The present invention relates to a blood carnitine-increasing agent.

### Background Art

Carnitine (3-hydroxy-4-trimethylammoniobutanoic acid) has various physiological actions with its L-isomer (L-carnitine: generally known as levocarnitine) as an active constituent. Known actions of carnitine include transportation of long-chain fatty acids into mitochondria matrices, maintenance of free CoA pools important for various metabolisms, an endogenous antidote action of binding to acyl groups of harmful acyl CoA to excrete the acyl CoA to the outside of cells and into urine, an antioxidant action, an anti-inflammation action, a biological membrane stabilizing action, and fibrilization suppressing action. In an adult, about 75% of the necessary amount of carnitine is supplied by meals, while the rest is biosynthesized in the body (liver, kidney and brain), and carnitine is present mainly in the tissues of the skeletal muscle, the heart, the liver and the like (Non Patent Literature 1).

Lack of carnitine in the body due to a decrease in intake or absorption of carnitine, a decrease in synthesis of carnitine in the body, an excessive loss of carnitine, an increase in excretion of free carnitine, an increase in excretion of acylcarnitine into urine, or the like leads to development of carnitine deficiency. It is known that carnitine deficiency develops in various clinical conditions. Such clinical conditions correspond to, for example, patients suspected to have carnitine deficiency in view of an age, an underlying disease or clinical condition, a muscle mass, a prescribed drug, a nutritional state, a dietary content and the like, such as congenital metabolic abnormality; patients receiving valproic acid administration (epileptic patients, psychiatric disease patients, patients after brain surgery, and the like); patients receiving peritoneal dialysis or hemodialysis therapy for kidney failure; Fanconi disease patients; patients receiving continuous renal replacement therapy (CRRT); patients receiving nutritional management by tubal feeding, total parenteral nutrition (TPN), modified milk powder free from some milk allergens, or the like; patients receiving administration of a pivoxil group containing antibacterial drug; patients receiving an anticancer agent administration (platinum-based preparation, anthracycline preparation, alkylating agent or the like); hepatic cirrhosis or hepatic failure patients; neuromuscular disease patients such as muscular dystrophy or amyotrophic lateral sclerosis (ALS) patients; severely handicapped children (persons); anorexia patients; aged persons; severe diseases; and undernutrition patients (Non Patent Literature 1).

The carnitine deficiency is classified broadly into primary carnitine deficiency developed by a decrease in incorporation of carnitine in muscular cells and renal tubules due to, for example, genetic abnormality mainly of a carnitine transporter (OCTN2), and secondary carnitine deficiency developed from other causes. The primary carnitine deficiency is severe carnitine deficiency also called systemic carnitine deficiency, where long-chain fatty acid metabolism necessary for ATP production is impaired, resulting in occurrence of sudden infant death syndrome, hypoketotic hypoglycemia, cardiomyopathy or the like (Non Patent Literature 1).

Examples of clinical symptoms/signs indicative of carnitine deficiency include disturbance of consciousness, convulsion, muscle hypotonia/muscular depression/severe cramps/severe fatigue, rhabdomyolysis, encephalopathy, vomiting induced by hunger/infection, frequent vomiting, delayed mental/motor development, a poor body weight gain, respiratory abnormality, cardiac enlargement/cardiomyopathy/cardiac hypofunction and sudden death (or family histories thereof), and the repetitive Reye-like syndrome. In addition, carnitine deficiency is suspected if general clinical test findings indicate hypoketotic hypoglycemia, metabolic acidosis, hyperammonemia, liver function abnormality (increased AST or ALT) and fatty liver, abnormality in blood gas analysis (pH, HCO³⁻, BE), electrolyte abnormality (Na, K, Ca, Cl), treatment-resistant anemia, or the like and other definite causes are denied. In addition to these symptoms, signs and findings, a blood carnitine two-fraction test or a result showing an improvement by supplementation of carnitine is used to make a diagnosis of carnitine deficiency (Non Patent Literature 1).

As a drug treatment method for carnitine deficiency, a carnitine supplementation therapy involving administration of a levocarnitine preparation is known. In the aspect of the carnitine supplementation therapy, an administration method, a dosage, an administration route and an administration period are determined by various factors. Examples of such factors include persistence of a primary disease or a cause of carnitine deficiency, whether in an acute phase or a chronic phase, a degree of severity, an individual patient combined factor, and urgency of treatment, and depending on each of the factors, an optimum dosage form of levocarnitine is selected from the group consisting of a tablet, an oral solution, an intravenous solution and the like (Non Patent Literature 1). However, a levocarnitine preparation is administered in a large amount of from 1.5 g to 3 g a day per adult, and oral administration at a high dose poses a risk of accumulating harmful metabolites such as trimethylamine through metabolism of the intestinal bacterial flora, and causes gastrointestinal side effects such as nausea, vomiting and diarrhea. Therefore, a treatment method involving supplementation of carnitine by a method other than administration of a levocarnitine preparation at high dose or a novel treating agent promoting absorption of a levocarnitine preparation are desired.

Diseases other than carnitine deficiency are known for which supplementation of carnitine may have a therapeutic effect. Examples of such diseases include diseases associated with a decrease in mitochondrial function which is associated with abnormal metabolism of carnitine, and diseases in which symptoms are relieved by an increase in blood carnitine level. In recent years, it has been pointed out that supplementation of carnitine to the skeletal muscle may have an effect of improving sarcopenia frailty (Non Patent Literatures 2 to 4).

For these diseases, supplementation of carnitine by administration of a levocarnitine preparation may have an effect, but it is still desirable to produce a novel medicament.

Meanwhile, Patent Literature 1 discloses that a compound of the following formula (1): 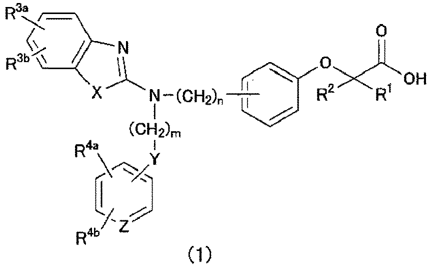 wherein R¹ and R² are the same or different, and each represent a hydrogen atom, a methyl group or an ethyl group; R^{3a}, R^{3b}, R^{4a} and R^{4b} are the same or different, and each represent a hydrogen atom, a halogen atom, a nitro group, a hydroxide group, a C₁₋₄ alkyl group, a trifluoromethyl group, a C₁₋₄ alkoxy group, a C₁₋₄ alkylcarbonyloxy group, a di-C₁₋₄ alkylamino group, a C₁₋₄ alkylsulfonyloxy group, a C₁₋₄ alkylsulfonyl group, a C₁₋₄ alkylsulfinyl group, or a C₁₋₄ alkylthio group, or R^{3a} and R^{3b} or R^{4a} and R^{4b} are bonded to each other to form an alkylenedioxy group; X represents an oxygen atom, a sulfur atom or N-R⁵ (R⁵ represents a hydrogen atom, a C₁₋₄ alkyl group, a C₁₋₄ alkylsulfonyl group, or a C₁₋₄ alkyloxycarbonyl group); Y represents an oxygen atom, a S(O)i group (l represents an integer of 0 to 2), a carbonyl group, a carbonylamino group, an aminocarbonyl group, a sulfonylamino group, an aminosulfonyl group, or a NH group; Z represents CH or N; n represents a number of 1 to 6; and m represents a number of 2 to 6, a salt thereof, or a solvate thereof has a selective PPARα activating action, and is useful as preventing and/or therapeutic drug for hyperlipidemia, arteriosclerosis, diabetes, complications of diabetes (diabetic renal disease and the like), inflammation, heart disease and the like in mammals including humans, which does not cause a body weight gain and obesity.

However, how such a compound acts on carnitine deficiency or a disease for which supplementation of carnitine may have a therapeutic effect is neither described nor suggested.

### Citation List

### Patent Literature

Patent Literature 1: WO 2005/023777

### Non Patent Literature

Non Patent Literature 1: "Carnitine", [online], March 29, 2021, U.S. Department of Health & Human Services National Institute of Health, [Search date: April 26, 2021], Internet <URL: https://ods.od.nih.gov/factsheets/Carnitine-HealthProfessional/>
Non Patent Literature 2: Hepatol Commun. 2(8) p906-918 (2018)
Non Patent Literature 3: Clinical nutrition 38(4) p523-31 (1983)
Non Patent Literature 4: Clinical Interventions in Aging p1675-1686 (2016)

### Summary of Invention

### Technical Problem

The present invention aims to provide a novel treating agent useful for treatment of carnitine deficiency or a disease for which supplementation of carnitine may have a therapeutic effect.

### Solution to Problem

For achieving the object, the present inventors have extensively conducted studies, and resultantly found that very surprisingly, a compound disclosed as Example 85 in Patent Literature 1 recited above, that is, (R)-2-[3-[[N-(benzoxazol-2-yl)-N-3-(4-methoxyphenoxy)propyl]aminomethyl]phenoxy]butyric acid (hereinafter, sometimes referred to as a "compound A" or "Pemafibrate") increases the concentration of free carnitine in blood by, for example, promoting synthesis of carnitine and absorption of carnitine, and is useful for prevention and/or treatment of carnitine deficiency or a disease for which supplementation of carnitine may have a therapeutic effect. In this way, the present invention has been completed.

That is, the present invention provides the following [1] to [41].
[1] A blood carnitine-increasing agent comprising (R)-2-[3-[[N-(benzoxazol-2-yl)-N-3-(4-methoxyphenoxy)propyl]aminomethyl]phenoxy]butyric acid, a salt thereof, or a solvate thereof as an active ingredient.
[2] The blood carnitine-increasing agent according to [1], further comprising levocarnitine as an active ingredient.
[3] A method for increasing blood carnitine, comprising administering an effective amount of (R)-2-[3-[[N-(benzoxazol-2-yl)-N-3-(4-methoxyphenoxy)propyl]aminomethyl]phenoxy]butyric acid, a salt thereof, or a solvate thereof to a patient in need thereof.
[4] The method for increasing blood carnitine according to [3], further comprising administering an effective amount of levocarnitine.
[5] Use of (R)-2-[3-[[N-(benzoxazol-2-yl)-N-3-(4-methoxyphenoxy)propyl]aminomethyl]phenoxy]butyric acid, a salt thereof, or a solvate thereof for producing a blood carnitine-increasing agent.
[6] The use according to [5] in combination with levocarnitine.
[7] (R)-2-[3-[[N-(benzoxazol-2-yl)-N-3-(4-methoxyphenoxy)propyl]aminomethyl]phenoxy]butyric acid, a salt thereof, or a solvate thereof for use in increasing blood carnitine.
[8] The (R)-2-[3-[[N-(benzoxazol-2-yl)-N-3-(4-methoxyphenoxy)propyl]aminomethyl]phenoxy]butyric acid, salt thereof, a solvate thereof according to [7], in combination with levocarnitine.
[9] A pharmaceutical composition for preventing and/or treating carnitine deficiency, comprising a therapeutically effective amount of (R)-2-[3-[[N-(benzoxazol-2-yl)-N-3-(4-methoxyphenoxy)propyl]aminomethyl]phenoxy]butyric acid, a salt thereof, or a solvate thereof.
[10] The pharmaceutical composition according to [9], further comprising levocarnitine as an active ingredient.
[11] An agent for preventing and/or treating carnitine deficiency, comprising (R)-2-[3-[[N-(benzoxazol-2-yl)-N-3-(4-methoxyphenoxy)propyl]aminomethyl]phenoxy]butyric acid, a salt thereof, or a solvate thereof as an active ingredient.
[12] The agent according to [11], further comprising levocarnitine as an active ingredient.
[13] A method for preventing and/or treating carnitine deficiency, comprising administering an effective amount of (R)-2-[3-[[N-(benzoxazol-2-yl)-N-3-(4-methoxyphenoxy)propyl]aminomethyl]phenoxy]butyric acid, a salt thereof, or a solvate thereof to a patient in need thereof.
[14] The prevention and/or treatment method according to [13], further comprising administering an effective amount of levocarnitine.
[15] Use of (R)-2-[3-[[N-(benzoxazol-2-yl)-N-3-(4-methoxyphenoxy)propyl]aminomethyl]phenoxy]butyric acid, a salt thereof, or a solvate thereof for preventing and/or treating carnitine deficiency.
[16] The use according to [15], in combination with levocarnitine.
[17] Use of (R)-2-[3-[[N-(benzoxazol-2-yl)-N-3-(4-methoxyphenoxy)propyl]aminomethyl]phenoxy]butyric acid, a salt thereof, or a solvate thereof for producing a pharmaceutical composition for preventing and/or treating carnitine deficiency.
[18] Use of (R)-2-[3-[[N-(benzoxazol-2-yl)-N-3-(4-methoxyphenoxy)propyl]aminomethyl]phenoxy]butyric acid, a salt thereof, or a solvate thereof for producing a pharmaceutical composition for preventing and/or treating carnitine deficiency, in combination with levocarnitine.
[19] (R)-2-[3-[[N-(benzoxazol-2-yl)-N-3-(4-methoxyphenoxy)propyl]aminomethyl]phenoxy]butyric acid, a salt thereof, or a solvate thereof for use in prevention and/or treatment of carnitine deficiency.
[20] A combination of (R)-2-[3-[[N-(benzoxazol-2-yl)-N-3-(4-methoxyphenoxy)propyl]aminomethyl]phenoxy]butyric acid, a salt thereof, or a solvate thereof and levocarnitine for use in prevention and/or treatment of carnitine deficiency.
[21] An agent for preventing and/or treating a disease associated with a decrease in mitochondrial function, comprising (R)-2-[3-[[N-(benzoxazol-2-yl)-N-3-(4-methoxyphenoxy)propyl]aminomethyl]phenoxy]butyric acid, a salt thereof, or a solvate thereof as an active ingredient.
[22] An agent for preventing and/or treating sarcopenia, comprising (R)-2-[3-[[N-(benzoxazol-2-yl)-N-3-(4-methoxyphenoxy)propyl]aminomethyl]phenoxy]butyric acid, a salt thereof, or a solvate thereof as an active ingredient.
[23] The agent according to [21] or [22], comprising levocarnitine as an active ingredient.
[24] A method for preventing and/or treating a disease associated with a decrease in mitochondrial function, comprising administering an effective amount of (R)-2-[3-[[N-(benzoxazol-2-yl)-N-3-(4-methoxyphenoxy)propyl]aminomethyl]phenoxy]butyric acid, a salt thereof, or a solvate thereof to a patient in need thereof.
[25] A method for preventing and/or treating sarcopenia, comprising administering an effective amount of (R)-2-[3-[[N-(benzoxazol-2-yl)-N-3-(4-methoxyphenoxy)propyl]aminomethyl]phenoxy]butyric acid, a salt thereof, or a solvate thereof to a patient in need thereof.
[26] The method according to [24] or [25], further comprising administering an effective amount of levocarnitine.
[27] Use of (R)-2-[3-[[N-(benzoxazol-2-yl)-N-3-(4-methoxyphenoxy)propyl]aminomethyl]phenoxy]butyric acid, a salt thereof, or a solvate thereof for producing an agent for preventing and/or treating a disease associated with a decrease in mitochondrial function.
[28] Use of (R)-2-[3-[[N-(benzoxazol-2-yl)-N-3-(4-methoxyphenoxy)propyl]aminomethyl]phenoxy]butyric acid, a salt thereof, or a solvate thereof for producing an agent for preventing and/or treating sarcopenia.
[29] The use according to [27] or [28], in combination with levocarnitine.
[30] (R)-2-[3-[[N-(benzoxazol-2-yl)-N-3-(4-methoxyphenoxy)propyl]aminomethyl]phenoxy]butyric acid, a salt thereof, or a solvate thereof for use in prevention and/or treatment of a disease associated with a decrease in mitochondrial function.
[31] (R)-2-[3-[[N-(benzoxazol-2-yl)-N-3-(4-methoxyphenoxy)propyl]aminomethyl]phenoxy]butyric acid, a salt thereof, or a solvate thereof for use in prevention and/or treatment of sarcopenia.
[32] The (R)-2-[3-[[N-(benzoxazol-2-yl)-N-3-(4-methoxyphenoxy)propyl]aminomethyl]phenoxy]butyric acid, a salt thereof, or a solvate thereof according to [30] or [31], in combination with levocarnitine.
[33] An agent for preventing and/or treating a disease in which symptoms are relieved by an increase in blood carnitine level, the agent comprising (R)-2-[3-[[N-(benzoxazol-2-yl)-N-3-(4-methoxyphenoxy)propyl]aminomethyl]phenoxy]butyric acid, a salt thereof, or a solvate thereof as an active ingredient.
[34] The agent according to [33], further comprising levocarnitine as an active ingredient.
[35] A method for preventing and/or treating a disease in which symptoms are relieved by an increase in blood carnitine level, the method comprising administering an effective amount of (R)-2-[3-[[N-(benzoxazol-2-yl)-N-3-(4-methoxyphenoxy)propyl]aminomethyl]phenoxy]butyric acid, a salt thereof, or a solvate thereof to a patient in need thereof.
[36] The method according to [35], further comprising administering an effective amount of levocarnitine.
[37] Use of (R)-2-[3-[[N-(benzoxazol-2-yl)-N-3-(4-methoxyphenoxy)propyl]aminomethyl]phenoxy]butyric acid, a salt thereof, or a solvate thereof for producing an agent for preventing and/or treating a disease in which symptoms are relieved by an increase in blood carnitine level.
[38] The use according to [37], in combination with levocarnitine.
[39] (R)-2-[3-[[N-(benzoxazol-2-yl)-N-3-(4-methoxyphenoxy)propyl]aminomethyl]phenoxy]butyric acid, a salt thereof, or a solvate thereof for use in prevention and/or treatment of a disease in which symptoms are relieved by an increase in blood carnitine level.
[40] The (R)-2-[3-[[N-(benzoxazol-2-yl)-N-3-(4-methoxyphenoxy)propyl]aminomethyl]phenoxy]butyric acid, a salt thereof, or a solvate thereof according to [39], in combination with levocarnitine.
[41] The blood carnitine-increasing agent according to [1] or [2], the agent according to any one of [11], [12] and [21] to [23], or the pharmaceutical composition according to [9] or [10], which is in the form of a kit comprising a preparation containing (R)-2-[3-[[N-(benzoxazol-2-yl)-N-3-(4-methoxyphenoxy)propyl]aminomethyl]phenoxy]butyric acid, a salt thereof, or a solvate thereof, and a preparation containing levocarnitine.

### Advantageous Effects of Invention

The present invention provides a novel medicament useful for prevention and/or treatment of carnitine deficiency or a disease for which supplementation of carnitine may have a therapeutic effect. According to the present invention, it is possible to provide a new option of prevention and/or treatment to a patient with carnitine deficiency or a disease for which supplementation of carnitine may have a therapeutic effect.

### Brief Description of Drawings

Figure 1 shows an amount of change in concentration of free carnitine in blood (µmol/L) in a fasting state when a compound A (0.2 to 0.8 mg a day) is administered to a dyslipidemia patient.
Figure 2 shows a change in concentration of free carnitine in blood (µmol/L) after a meal when the compound A (0.2 to 0.8 mg a day) is administered to a dyslipidemia patient.
Figure 3 shows a change in concentration of free carnitine in blood (µmol/L) in a fasting state when the compound A (0.4 mg a day) or placebo is administered to a NAFLD patient.

### Description of Embodiments

(R)-2-[3-[[N-(benzoxazol-2-yl)-N-3-(4-methoxyphenoxy)propyl]aminomethyl]phenoxy]butyric acid (compound A) for use in the present invention is represented by the following chemical formula (A):

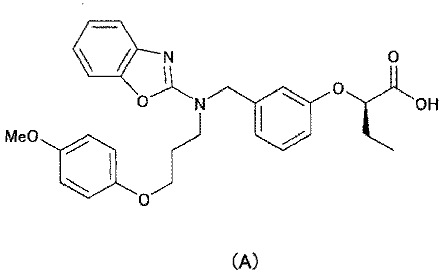

The compound A can be produced in accordance with a method described in, for example, WO 2005/023777. It is also possible to formulate the compound A in accordance with a method described in literature. Further, a preparation containing the compound A has been approved as an agent for treating hyperlipemia "Parmodia (registered trademark) Tab." in Japan, and it is also possible to use the "Parmodia Tab.".

In an embodiment of the present invention, it is also possible to use a salt or a solvate of the compound A. The salt and the solvate can be produced by a conventional method. The salt of the compound A is not particularly limited as long as it is pharmaceutically acceptable, and examples thereof include alkali metal salts such as sodium salts and potassium salts; alkaline earth metal salts such as calcium salts and magnesium salts; organic base salts such as ammonium salts and trialkylamine salts; mineral acid salts such as hydrochloric acid salts and sulfuric acid salts; and organic acid salts such as acetic acid salts. Examples of the solvate of the compound A or a salt thereof include hydrates and alcohol adducts (e.g., ethanol adducts).

In an aspect of the present invention, the compound A, a salt thereof, or a solvate thereof may be used in combination with levocarnitine for use in carnitine supplementation therapy from the viewpoint of increasing the blood carnitine level. Specifically, in place of levocarnitine or in combination with levocarnitine, the compound A, a salt thereof, or a solvate thereof can be administered to a patient with levocarnitine-resistant carnitine deficiency which is not improved even by administering levocarnitine, or a disease for which supplementation of carnitine may have a therapeutic effect.

In the present specification, the "levocarnitine" refers to L-carnitine, and its commercially available preparation can be used. Examples of the preparation containing levocarnitine include L-Cartin (registered trademark) FF tablets 100 mg, L-Cartin FF tablets 250 mg, L-Cartin FF oral solution 10%, L-Cartin FF oral solution 10% single-dose package 5 mL, L-Cartin FF oral solution 10% single-dose package 10 mL and L-Cartin FF injection 1 000 mg (each from Otsuka Pharmaceutical Co., Ltd.), and "Levocarnitine hydrochloride tablets 100 mg/300 mg" sold by various companies as generic medications. It is also possible to use CARNITOR Tablets, CARNITOR Oral Solution and the like approved in foreign countries.

As shown in Examples described later, the compound A increases the concentration of free carnitine in blood. Since the concentrations of free carnitine in blood in a fasting state and after a meal are increased by administration of the compound A, the compound A is considered to have an action of accelerating synthesis of carnitine in the body and an action of promoting absorption of carnitine from food.

Therefore, the compound A, a salt thereof, or a solvate thereof may be an active ingredient of the blood carnitine-increasing agent. The compound A, a salt thereof, or a solvate thereof is useful for increasing the concentration of free carnitine in blood to prevent and/or treat carnitine deficiency, or a disease for which supplementation of carnitine may have a therapeutic effect. Examples of the disease for which supplementation of carnitine may have a therapeutic effect include diseases associated with a decrease in mitochondrial function which is related to abnormal metabolism of carnitine, and diseases in which symptoms are relieved by an increase in blood carnitine level.

In the present specification, unless otherwise expressly specified, the term "blood carnitine-increasing agent" refers to, for example, a medicament which, when applied, increases the blood carnitine level to relieve or improve the symptoms of, for example, carnitine deficiency, diseases associated with a decrease in mitochondrial function, or diseases in which symptoms are relieved by an increase in blood carnitine level.

In the present specification, unless otherwise expressly specified, the term "carnitine deficiency" refers to a state in which there is a lack of carnitine necessary in living organisms, and examples thereof include a state in which the concentration of free carnitine is less than 20 µmol/L in a blood carnitine test and a state in which symptoms are relieved or improved by carnitine supplementation therapy, while whether the disease occurs or not is usually determined on the basis of diagnostic criteria. In the present invention, "being extremely likely to develop carnitine deficiency" is also regarded as a target of prevention and/or treatment of "carnitine deficiency".

The diagnosis of carnitine deficiency is performed by a combination of a "blood carnitine test", "clinical symptoms/clinical signs" commonly observed in carnitine deficiency, and "general clinical test findings". "Therapeutic diagnosis" is also performed if absolutely necessary.

In the "blood carnitine test", the standard value of the free carnitine concentration is 36 µmol/L or more and 74 µmol/L or less. A free carnitine concentration of less than 20 µmol/L (< 20 µmol/L) leads to being diagnosed as "having carnitine deficiency" or "being ready to have carnitine deficiency". A free carnitine concentration of 20 µmol/L or more and less than 36 µmol/L (20 ≤ concentration of free carnitine in blood < 36 µmol/L) (that is, a borderline state in which the free carnitine level is below the standard value, but is not extremely low) or an acyl carnitine/free carnitine ratio of more than 0.4 (> 0.4) leads to being diagnosed as "being extremely likely to develop carnitine deficiency".

In the present specification, the "prevention and/or treatment of carnitine deficiency" is intended to ensure that for example, improving or promoting intake or absorption of carnitine, which has decreased, improving or increasing synthesis of carnitine in the body, which has decreased, suppressing an excessive loss of carnitine, suppressing an increase in excretion of free carnitine, suppressing an increase in excretion of acylcarnitine in urine, or the like, deficient carnitine is brought back to or close to the standard value (36 µmol/L ≤ concentration of free carnitine in blood ≤ 74 µmol/L) to secure free carnitine as a carrier of long-chain fatty acids, and acyl groups of accumulated toxic acyl CoA are received, and discharged as acylcarnitine to the outside of mitochondria, the outside of cells and the outside of the body to maintain free CoA pools necessary for various metabolisms.

In the present specification, the term "disease associated with a decrease in mitochondrial function" means a clinical condition caused by a decrease in energy production due to a decrease in mitochondrial function. Examples of the clinical condition include central neurological diseases such as convulsion, myoclonus, deconditioning, stroke-like symptoms, decreased intelligence, migraine headache, psychological symptoms, dystonia and myelopathy, skeletal muscular diseases such as muscular depression, easy fatigability, hyperCKemia, myopathy and sarcopenia, heart diseases such as conduction disturbance, Wolf-Parkinson-White syndrome, cardiac myopathy and pulmonary hypertension, ocular diseases such as optic nerve atrophy, external ophthalmoplegia and retinal pigment degeneration, hepatic diseases such as liver dysfunction and hepatic failure, renal diseases such as Fanconi syndrome, renal tubular dysfunction, glomerular lesion and urine myoglobin, pancreatic diseases such as diabetes and external secretion failure, blood diseases such as sideroblastic anemia and pancytopenia, inner ear diseases such as sensory deafness, intestinal diseases such as diarrhea and constipation, dermatological diseases such as decreased sweating and excessive hair growth, and endocrine diseases such as short stature and hypocalcemia.

In the present specification, the term "prevention and/or treatment of a disease associated with a decrease in mitochondrial function" means that, for example, in the above-described "disease associated with a decrease in mitochondrial function", deficient carnitine is brought back to or close to the standard value (36 µmol/L ≤ concentration of free carnitine in blood ≤ 74 µmol/L) to achieve relief, remission or healing of symptoms. The standard value is not necessarily satisfied as long as achievement of relief, remission or healing of symptoms is shown by findings or diagnosis.

In the present specification, the term "disease in which symptoms are relieved by an increase in blood carnitine level" means a clinical condition caused by a decrease in blood carnitine level. Examples of the clinical condition include disturbance of consciousness, convulsion, muscle hypotonia/muscular depression/severe cramps/severe fatigue, rhabdomyolysis, encephalopathy, vomiting induced by hunger/infection, frequent vomiting, delayed mental/motor development, a poor body weight gain, respiratory abnormality, cardiac enlargement/cardiomyopathy/cardiac hypofunction and sudden death (or family histories thereof), and the repetitive Reye-like syndrome.

In the present specification, the term "prevention and/or treatment of a disease in which symptoms are relieved by an increase in blood carnitine level" means that for example, in the above "disease in which symptoms are relieved by an increase in blood carnitine level", deficient carnitine is brought back to or close to the standard value (36 µmol/L ≤ concentration of free carnitine in blood ≤ 74 µmol/L) to achieve relief, remission or healing of symptoms. The standard value is not necessarily satisfied as long as achievement of relief, remission or healing of symptoms is shown by findings or diagnosis.

In an aspect of the present invention, for using the compound A, a salt thereof, or a solvate thereof, and levocarnitine as a medicament, a dosage form such as a tablet, a capsule, a granule, a powder, a lotion, an ointment, an injection or a suppository can be obtained by using other pharmaceutically acceptable carriers if necessary. Such a preparation can be produced by a known method.

Examples of the pharmaceutically acceptable carrier include excipients, disintegrants, binders, lubricants, plasticizers, fluidizers, diluents, solubilizers, suspending agents, tonicity agents, pH adjusters, buffering agents, stabilizers, coatings, colorants, flavor improvers, and odor improvers.

When the compound A, a salt thereof, or a solvate thereof and levocarnitine are used in combination, the compound A, a salt thereof, or a solvate thereof and levocarnitine may be formulated collectively in one dosage form (that is, formulated as a combination agent). A kit may also be provided in which a preparation containing the compound A, a salt thereof, or a solvate thereof and a preparation containing levocarnitine can be used in parallel or separately with an interval therebetween. When the preparations are administered independently, whichever of the preparations may be administered first.

When the compound A, a salt thereof, or a solvate thereof and levocarnitine are used in combination, the dose or combination ratio is not particularly limited as long as the effect is exhibited. For example, the mass ratio between the compound A (free form) and levocarnitine is in the range of 1 : 1 000 000 to 1 . 100.

In an aspect of the present invention, the compound A, a salt thereof, or a solvate thereof may be administered orally or parenterally, and oral administration is preferable. The therapeutically effective amount and number of doses of the compound A, a salt thereof, or a solvate thereof vary depending on a body weight, an age, a sex, symptoms of a patient, and the like, and can be appropriately set by those skilled in the art. For example, for an adult, the compound A can be normally administered at 0.05 to 0.8 mg per day in one to three divided doses, and is administered preferably at 0.1 to 0.4 mg per day once or in two divided doses, more preferably at 0.2 to 0.4 mg per day once or in two divided doses.

For the dosage of levocarnitine, it is provided in a package insert that for an adult, normally, levocarnitine is orally administered at 1.5 to 3 g a day in three divided doses.

An object to which the medicament of the present invention is administered is a human in need of an increase in carnitine, preferably a patient with carnitine deficiency, or a patient with a disease for which supplementation of carnitine may have a therapeutic effect (a patient with a disease associated with a decrease in mitochondrial function, a patient with a disease in which symptoms are relieved by an increase in blood carnitine level, or the like). It is also possible to perform the administration on a patient receiving a medicament which causes carnitine deficiency, such as a patient receiving valproic acid, a patient receiving pivoxil group-containing antibacterial drug, or a patient receiving an anticancer agent. For these applications, levocarnitine may be added in addition to the compound A, a salt thereof, or a solvate thereof as described above.

Hereinafter, the present invention will be described in further detail by way of Examples, but these Examples should not be construed to limit the present invention.

### Examples

### Example 1: Study on action of compound A on blood carnitine (dyslipidemia patient)

A study was conducted on an action on blood carnitine in oral administration of the compound A to a dyslipidemia patient at 0.2 to 0.8 mg a day for 4 weeks. For the administration of the compound A, an IR preparation (0.2 mg/day, twice a day), 0.4 mg of a CR preparation (once a day) and 0.8 mg of a CR preparation (once a day) were used, and the administration was performed before or after a meal.

### Example 2: Study on action of compound A on blood carnitine (NAFLD patient)

A study was conducted on an action on blood carnitine in oral administration of the compound A to a non-alcoholic fatty liver disease (NAFLD) patient at 0.4 mg a day for 48 weeks. For the administration of the compound A, an IR preparation (0.4 mg/day, twice a day) was used, and the administration was performed in a fasting state. A comparison was made with placebo administration.

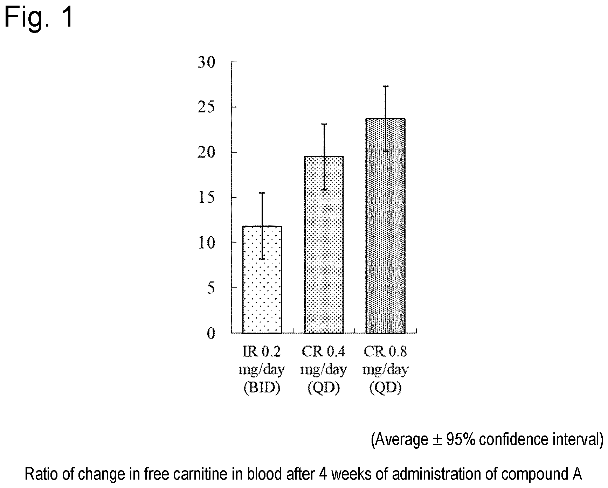

Table 1 and Figure 1 show a ratio of change in concentration of free carnitine in blood in a fasting state in administration of the compound A to the dyslipidemia patient for 4 weeks with respect to the value before administration (at 0 weeks) in Example 1.

Administration of the compound A increased the concentration of free carnitine in blood in a dose-dependent manner. The compound A was revealed to have an action of increasing the concentration of free carnitine in blood in a fasting state.

**[Table 1]**

| Ratio of change in free carnitine after administration for 4 weeks with respect to value before administration of compound A (at 0 weeks) | | | |
|---|---|---|---|
| Administration group | Number of cases | Ratio of change | 95% Confidence interval |
| IR 0.2 mg/day | 40 | 11.82 | 8.18, 15.46 |
| CR 0.4 mg/day | 40 | 19.5 | 15.86, 23.14 |
| CR 0.8 mg/day | 40 | 23.71 | 20.07, 27.35 |

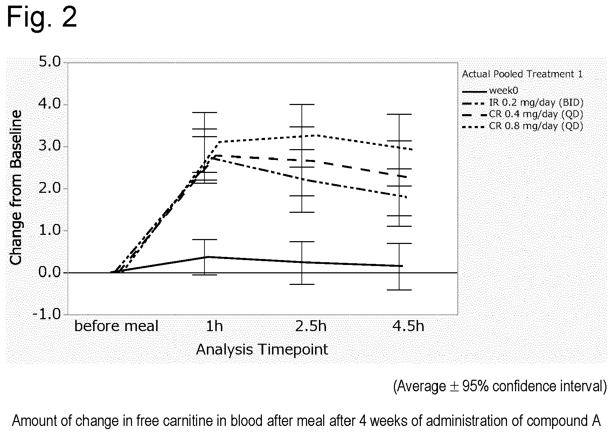

Table 2 and Figure 2 show a process of an amount of change in free carnitine in blood after meal (after intake of high-fat food) with respect to a value before meal in administration of the compound A to the dyslipidemia patient for 4 weeks in Example 1.

Administration of the compound A increased the concentration of free carnitine in blood after meal as compared to the concentration before meal. The compound A was revealed to have an action of increasing the concentration of free carnitine in blood after meal.

**[Table 2]**

| 1 h | | | 2.5 h | | | 4.5 h | | | |
|---|---|---|---|---|---|---|---|---|---|
| | N | Average | 95% Confidence interval | N | Average | 95% Confidence interval | N | Average | 95% Confidence interval |
| Before administration of compound A (0 weeks) | 60 | 0.37 | -0.05, 0.80 | 60 | 0.24 | -0.27, 0.74 | 60 | 0.15 | -0.41, 0.71 |
| IR 0.2 mg/day (BID) after administration for 4 weeks | 40 | 2.72 | 2.21, 3.24 | 40 | 2.19 | 1.44, 2.93 | 40 | 1.79 | 1.11, 2.48 |
| CR 0.4 mg/day (QD) after administration for 4 weeks | 40 | 2.78 | 2.13, 3.43 | 40 | 2.65 | 1.83, 3.48 | 40 | 2.25 | 1.36, 3.14 |
| CR 0.8 mg/day (QD) after administration for 4 weeks | 39 | 3.11 | 2.40, 3.81 | 39 | 3.26 | 2.52, 4.00 | 39 | 2.92 | 2.06, 3.77 |

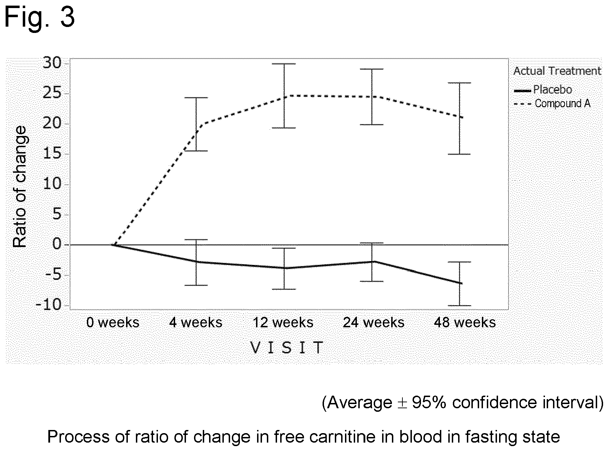

Table 3 and Figure 3 show a process of a ratio of change in concentration of free carnitine in blood in a fasting state in administration of the compound A (IR 0.4 mg/day) to the NAFLD patient for 48 weeks with respect to the value before administration (at 0 weeks) in Example 2.

Administration of the compound A increased the concentration of free carnitine in blood in a fasting state as compared to placebo administration. It was revealed that the concentration of free carnitine in blood was increased by administration of the compound A, and the increasing action reached the maximum value at 12 weeks of administration.

**[Table 3]**

| | Placebo | | Compound A | | | | | |
|---|---|---|---|---|---|---|---|---|
| | 4 weeks | 12 weeks | 24 weeks | 48 weeks | 4 weeks | 12 weeks | 24 weeks | 48 weeks |
| N | 60 | 60 | 59 | 59 | 58 | 57 | 57 | 55 |
| Average | -2.9 | -3.9 | -2.8 | -6.4 | 20.0 | 24.7 | 24.5 | 20.9 |
| 95% Confidence interval | -6.62, 0.83 | -7.26, -0.53 | -5.92, 0.29 | -9.97, -2.83 | 15.5, 24.4 | 19.40, 29.96 | 19.91, 29.06 | 15.08, 26,79 |

With reference to Tables 1 to 3 and Figures 1 to 3, administration of the compound A increased the concentrations of free carnitine both in blood in a fasting state and after meal , and the compound A was considered to have an action of accelerating synthesis of carnitine in the body, an action of promoting absorption of carnitine from food, and the like. Therefore, the compound A was shown to be useful as an agent for preventing and/or treating carnitine deficiency, or a disease for which supplementation of carnitine may have a therapeutic effect.

### Industrial Applicability

The present invention, which has been completed upon the discovery that the compound A has an action of increasing the concentration of free carnitine in blood in a fasting state and after a meal, is beneficial as a medicament for preventing and/or treating carnitine deficiency, or a disease for which supplementation of carnitine may have a therapeutic effect.

## Claims

1. A blood carnitine-increasing agent comprising (R)-2-[3-[[N-(benzoxazol-2-yl)-N-3-(4-methoxyphenoxy)propyl]aminomethyl]phenoxy]butyric acid, a salt thereof, or a solvate thereof as an active ingredient.

2. The blood carnitine-increasing agent according to claim 1, further comprising levocarnitine as an active ingredient.

3. A pharmaceutical composition for preventing and/or treating carnitine deficiency, comprising a therapeutically effective amount of (R)-2-[3-[[N-(benzoxazol-2-yl)-N-3-(4-methoxyphenoxy)propyl]aminomethyl]phenoxy]butyric acid, a salt thereof, or a solvate thereof.

4. The pharmaceutical composition according to claim 2, further comprising levocarnitine as an active ingredient.

5. A preventing and/or treating agent for a disease associated with a decrease in mitochondrial function, comprising (R)-2-[3-[[N-(benzoxazol-2-yl)-N-3-(4-methoxyphenoxy)propyl]aminomethyl]phenoxy]butyric acid, a salt thereof, or a solvate thereof as an active ingredient.

6. A preventing and/or treating agent for sarcopenia, comprising (R)-2-[3-[[N-(benzoxazol-2-yl)-N-3-(4-methoxyphenoxy)propyl]aminomethyl]phenoxy]butyric acid, a salt thereof, or a solvate thereof as an active ingredient.

7. The preventing and/or treating agent according to claim 5 or 6, comprising levocarnitine as an active ingredient.

8. Use of (R)-2-[3-[[N-(benzoxazol-2-yl)-N-3-(4-methoxyphenoxy)propyl]aminomethyl]phenoxy]butyric acid, a salt thereof, or a solvate thereof for producing a blood carnitine-increasing agent.

9. Use of (R)-2-[3-[[N-(benzoxazol-2-yl)-N-3-(4-methoxyphenoxy)propyl]aminomethyl]phenoxy]butyric acid, a salt thereof, or a solvate thereof for producing a pharmaceutical composition for preventing and/or treating carnitine deficiency.

10. Use of (R)-2-[3-[[N-(benzoxazol-2-yl)-N-3-(4-methoxyphenoxy)propyl]aminomethyl]phenoxy]butyric acid, a salt thereof, or a solvate thereof for producing a preventing and/or treating agent for a disease associated with a decrease in mitochondrial function.

11. Use of (R)-2-[3-[[N-(benzoxazol-2-yl)-N-3-(4-methoxyphenoxy)propyl]aminomethyl]phenoxy]butyric acid, a salt thereof, or a solvate thereof for producing a preventing and/or treating agent for sarcopenia.

12. The use according to any one of claims 8 to 11, in combination with levocarnitine.

13. (R)-2-[3-[[N-(benzoxazol-2-yl)-N-3-(4-methoxyphenoxy)propyl]aminomethyl]phenoxy]butyric acid, a salt thereof, or a solvate thereof for use in increasing blood carnitine.

14. (R)-2-[3-[[N-(benzoxazol-2-yl)-N-3-(4-methoxyphenoxy)propyl]aminomethyl]phenoxy]butyric acid, a salt thereof, or a solvate thereof for use in prevention and/or treatment of carnitine deficiency.

15. (R)-2-[3-[[N-(benzoxazol-2-yl)-N-3-(4-methoxyphenoxy)propyl]aminomethyl]phenoxy]butyric acid, a salt thereof, or a solvate thereof for use in prevention and/or treatment of a disease associated with a decrease in mitochondrial function.

16. (R)-2-[3-[[N-(benzoxazol-2-yl)-N-3-(4-methoxyphenoxy)propyl]aminomethyl]phenoxy]butyric acid, a salt thereof, or a solvate thereof for use in prevention and/or treatment of sarcopenia.

17. The (R)-2-[3-[[N-(benzoxazol-2-yl)-N-3-(4-methoxyphenoxy)propyl]aminomethyl]phenoxy]butyric acid, a salt thereof, or a solvate thereof according to any one of claims 13 to 16, in combination with levocarnitine.

18. A method for increasing blood carnitine, comprising administering an effective amount of (R)-2-[3-[[N-(benzoxazol-2-yl)-N-3-(4-methoxyphenoxy)propyl]aminomethyl]phenoxy]butyric acid, a salt thereof, or a solvate thereof to a patient in need thereof.

19. A method for preventing and/or treating carnitine deficiency, comprising administering an effective amount of (R)-2-[3-[[N-(benzoxazol-2-yl)-N-3-(4-methoxyphenoxy)propyl]aminomethyl]phenoxy]butyric acid, a salt thereof, or a solvate thereof to a patient in need thereof.

20. A method for preventing and/or treating a disease associated with a decrease in mitochondrial function, comprising administering an effective amount of (R)-2-[3-[[N-(benzoxazol-2-yl)-N-3-(4-methoxyphenoxy)propyl]aminomethyl]phenoxy]butyric acid, a salt thereof, or a solvate thereof to a patient in need thereof.

21. A method for preventing and/or treating sarcopenia, comprising administering an effective amount of (R)-2-[3-[[N-(benzoxazol-2-yl)-N-3-(4-methoxyphenoxy)propyl]aminomethyl]phenoxy]butyric acid, a salt thereof, or a solvate thereof to a patient in need thereof.

22. The method according to any one of claims 18 to 21, further comprising administering an effective amount of levocarnitine.
